# EUROPÄISCHE PATENTSCHRIFT

(11) **EP 3 938 031 B1**
(45) Veröffentlichungstag und Bekanntmachung des Hinweises auf die Patenterteilung: **01.03.2023**
(21) Anmeldenummer: 20710830.9
(22) Anmeldetag: 02.03.2020
(51) Int. Cl.: A61N 1/375

(54) **VERFAHREN ZUM HERSTELLEN EINES MEDIZINISCHEN IMPLANTATS**
METHOD FOR PRODUCING A MEDICAL IMPLANT
PROCÉDÉ DE FABRICATION D'UN IMPLANT MÉDICAL

(30) Priorität: 11.03.2019 DE 102019203273
(43) Veröffentlichungstag der Anmeldung: 19.01.2022
(73) Patentinhaber: Neuroloop GmbH, 79110 Freiburg (DE)
(72) Erfinder: KIMMIG, Fabian, 79108 Freiburg (DE); BORETIUS, Tim, 79108 Freiburg (DE); ADAMI, Florian, 79108 Freiburg (DE)
(74) Vertreter: Rösler, Uwe
(86) Internationale Anmeldenummer: PCT/EP2020/055465
(87) Internationale Veröffentlichungsnummer: WO 2020/182525

(56) Entgegenhaltungen:
- EP-A2- 2 082 780
- WO-A1-2017/029615
- DE-A1- 4 131 259
- US-A1- 2012 193 119

## Beschreibung

### Technisches Gebiet

Die Erfindung bezieht sich auf ein Verfahren zum Herstellen eines medizinischen Implantats, umfassend ein Kopfteil, das wenigstens eine in Art einer elektrischen Steckkontaktbuchse ausgebildete, sacklochartige Ausnehmung besitzt, längs der wenigstens ein elektrisch leitendes Kontaktelement angeordnet ist, sowie ein mit dem Kopfteil fest verbundenes Versorgungsteil, das wenigstens eine elektrische Komponente, vorzugsweise in Form eines Mikrocontrollers und/oder einer elektrische Energiequelle umfasst, die mit dem wenigstens einen elektrisch leitenden Kontaktelement über wenigstens eine elektrische Leiterstruktur elektrisch verbunden sind.

### Stand der Technik

Implantierbare medizinische Vorrichtungen zum Zwecke der elektrischen Stimulation lokaler intrakorporaler Bereiche, kurz implantierbare Impulsgeber, (IPG) beispielsweise für herztherapeutische Defribillations-, Schrittmacher- sowie Resynchronisationsanwendungen, für neurostimulationstherapeutische Maßnahmen, wie bspw. Rückenmarstimulation, Hirnstimulation oder Vagusnervstimulation, um nur einige zu nennen, weisen in aller Regel eine in sich abgeschlossenes Gehäuse auf, in dem Komponenten zur elektrischen Pulsgeneration enthalten sind, so zumindest eine elektrische Energiequelle und eine mit dieser verbundenen elektrischen Schaltungsstruktur. Zudem schließt an das Gehäuse ein sogenanntes Kopfteil an, in dem eine mit der Energiequelle bzw. der elektrischen Schaltungsstruktur elektrisch verbundene, elektrische Kontaktanordnung enthalten ist, in die eine fluiddicht mit dem Kopfteil abschließende Steckeranordnung einfügbar ist, die mit elektrischen Zuund Ableitungen zum Zwecke der intrakorporalen lokalen Applikation der elektrischen Stimulationssignale sowie gegebenenfalls der Zuführung intrakorporal lokal abgegriffener elektrischer Signale zur Gehäuseseitig vorhandenen elektrischen Schaltungsstruktur, kontaktiert ist.

In der Druckschrift EP 2 134 418 B1 ist ein gattungsgemäßes Kopfteil einer implantierbaren medizinischen Vorrichtung beschrieben, das zwei längs einer Fügenaht zusammenfügbare Kopfteilgehäusehälften umfasst, in die jeweils in serieller Abfolge durch Zwischenwände beabstandet, halbzylinderförmige Ausnehmungen eingebracht sind, in die in jeweils seriell abwechselnder Reihenfolge elektrisch leitende Kontaktringelemente sowie elektrisch isolierende Dichtungsringe eingesetzt sind. Das aus den zwei Kopfteilgehäusehälften zusammengefügte Kopfteil umfasst somit eine Anordnung koaxial zueinander ausgerichteter und elektrisch isolierter Kontaktringelemente, zu deren elektrischen Kontaktierung ein seitlicher Zugang im Kopfteil vorgesehen ist, durch den eine elektrische Steckeranordnung in einen von sämtlichen ringförmigen Kontaktringelementen umfassten Hohlraum fluiddicht einfügbar ist.

Die Druckschrift DE 10 2012 010 901 A1 offenbart ein Verfahren zum Positionieren und Halten von elektrischen Kontakten und Dichtungen innerhalb eines Kopfteils zur elektrischen Kontaktierung an eine medizinische implantierbare Vorrichtung. In das aus einem bioverträglichen und elektrisch isolierenden Material bestehende Kopfteilgehäuse ist einseitig eine sacklochartige Bohrung eingebracht, in der in abwechselnder Reihenfolge elektrisch leitende Kontaktringe und ringförmige Dichtelemente eingebracht sind, die gemeinsam einen Hohlraum umfassen, in den eine stiftförmige Steckeranordnung einfügbar ist. Jeder der einzelnen ringförmigen Kontaktringe ist innerhalb des Kopfteils ist über eine elektrische Verbindungsleitung mit innerhalb des Gehäuses der medizinische implantierbare Vorrichtung befindlichen elektrischen Komponenten verbunden.

In der Druckschrift DE 20 2013 012 073 U1 ist eine Steckerbohrung-Modulbaugruppe offenbart, zu deren Montage eine Anzahl von Kontaktringen und Dichtelementen in abwechselnder Reihenfolge längs eines stiftförmigen Montagewerkzeuges angeordnet sind. Mittels einer Spannvorrichtung werden sämtliche längs des Montagewerkzeuges aufsitzenden Kontaktringe und Dichtelemente unter Aufbringung einer axialen Fügekraft gegeneinander verspannt. Zum Zwecke der Konservierung der Fügekraft dient ein mittels einer Madenschraube axialfest auf dem Montagewerkzeug aufsitzendes Hülsenelement, das zusammen mit einem endseitigen Montagewerkzeugkopf die Anordnung aus Kontaktringen und Dichtelementen axial beidseitig begrenzt. In diesem verspannten Zustand erfolgt ein Vergießen der Anordnung mit einer aushärtbaren Vergußmasse, die im erstarrten Zustand die Fügekraft aufnimmt.

Herstellungsverfahren für ein medizinisches Implantat sind aus den Druckschriften US 2016 / 0 166 825 A1 sowie US 2008/ 0 033 500 A1 bekannt. In beiden Fällen sind der Versorgungsteil und das Kopfelement zumindest in einem finalen Herstellungsschritt von einer einstückigen Vergußmasse umgossen.

Die Druckschrift US 2003 / 0 144 707 A1 offenbart eine implantierbare medizinische Vorrichtung mit einer Oberflächenkontaktanordnung.

Die Druckschrift DE 10 2017 222 364 A1 beschreibt ein Verfahren zum Herstellen eines Kopfteils einer implantierbaren medizinischen Vorrichtung, mit einer Steckkontaktbuchse, in der eine serielle Abfolge von ringförmigen Kontaktelementen und elektrisch isolierende Dichtungsringe angeordnet ist. Zur mechanisch gegenseitigen Verspannung der Ringelemente dient ein Montagewerkzeug, das nach Fertigstellung der medizinischen Vorrichtung entnommen wird, wobei die kraftbeaufschlagte, gegenseitige Lagerung der Ringelemente von der verfestigten Kunststoffmatrix des Kopfteils aufgenommen wird.

Die Druckschrift EP 2 082 780 A2 offenbart ein Verfahren zum Herstellen eines medizinischen Implantats, umfassend ein Kopfteil, das wenigstens eine sacklochartige Ausnehmung in Art einer elektrischen Steckkontaktbuchse besitzt, längs der wenigstens ein elektrisch leitendes Kontaktelement angeordnet ist, sowie ein mit dem Kopfteil fest verbundenes Versorgungsteil, das wenigstens eine elektrische Komponente umfasst, die mit dem wenigstens einen elektrisch leitenden Kontaktelement über wenigstens eine elektrische Leiterstruktur (8) elektrisch verbunden sind.

### Darstellung der Erfindung

Der Erfindung liegt die Aufgabe zugrunde ein Verfahren zum Herstellen eines medizinischen Implantats, umfassend ein Kopfteil, das wenigstens eine in Art einer elektrischen Steckkontaktbuchse ausgebildete, sacklochartige Ausnehmung besitzt, längs der wenigstens ein elektrisch leitendes Kontaktelement angeordnet ist, sowie ein mit dem Kopfteil fest verbundenes Versorgungsteil, das wenigstens eine elektrische Komponente, vorzugsweise in Form eines Mikrocontrollers und/oder einer elektrische Energiequelle umfasst, die mit dem wenigstens einen elektrisch leitenden Kontaktelement über wenigstens eine elektrische Leiterstruktur elektrisch verbunden sind, derart weiterzubilden, so dass der verfahrenstechnische sowie auch zeit- und kostenspezifische Aufwand für die Herstellung sowohl individuell konfektionierter als auch von in großer Stückzahl gefertigter Implantate signifikant reduziert werden soll. Von besonderem Interesse ist es überdies, dass die Fertigungsqualität sowie die Fluiddichtigkeit und die damit verbundene Lebensdauer der Implantate höchsten Ansprüchen genügen sollen. Da es aus verfahrenstechnische Gründen bislang erforderlich ist das Kopfteil und das Versorgungsteil in jeweils getrennten Bauprozessen zu fertigen, gilt dem Fügen von Kopfteil und Versorgungsteil besondere Aufmerksamkeit, um unter Maßgabe der vorstehenden Fertigungsansprüchen eine dauerhaft fluiddichte Fügeverbindung realisieren zu können.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der Beschreibung, insbesondere unter Bezugnahme auf die illustrierten Ausführungsbeispiele zu entnehmen.

Das lösungsgemäße Verfahren zum Herstellen eines medizinischen Implantats gemäß den Merkmalen des Oberbegriffes des Anspruches 1 setzt sich aus den folgenden Verfahrensschritten zusammen.

Von zentraler Bedeutung bei der Herstellung gattungsgemäßer medizinischer Implantate ist die Ausbildung der Fügeverbindung zwischen Kopfteil und Versorgungsteil, die lösungsgemäß stoffschlüssig, d.h. monolithisch ausgebildet wird und somit für einen dauerhaften fluiddichten Fügeverbund zwischen Kopf- und Versorgungsteil sorgt. Unabhängig von der speziellen technischen Ausgestaltung des Kopf- sowie auch Versorgungsteils, die beide im Wege eines Gießverfahrens mit einer biokompatiblen Vergussmasse umhüllt werden, sind sowohl das Kopf- als auch das Versorgungsteil stoffschlüssig an zwei sich gegenüberliegenden Oberseiten einer Verbindungsplatte gefügt, die im weiteren als Fixierplatte bezeichnet wird und aus einem aushärtbaren Vergußmaterial besteht, mit dem sowohl das Kopf- und als Versorgungsteil umgossen sind.

Zum Zwecke der elektrischen Verbindung des wenigstens einen im Kopfteil enthaltenen, elektrisch leitenden Kontaktelementes mit dem im Versorgungsteil vorzugsweise enthaltenen Mikrocontroller sowie der elektrischen Energiequelle gilt es, die wenigstens eine elektrische Leiterstruktur durch die das Kopfteil mit dem Versorgungsteil verbindende Fixierplatte zu führen.

Das lösungsgemäße Verfahren sieht zunächst die Herstellung der über eine Plattenober- und -unterseite verfügenden Fixierplatte vor, die als Halbzeug im Wege eines Gießverfahrens unter Verwendung einer aushärtbaren Vergussmasse gewonnen wird. Während des Gießprozesses wird wenigstens eine elektrische Leiterstruktur, vorzugsweis in Form eines Drahtabschnittes, orthogonal zu der sich ausbildenden Plattenober- und-unterseite der Fixierplatte orientiert und angeordnet, so dass die Leiterstruktur nach dem Aushärten der Vergussmasse fest mit der Fixierplatte verbunden ist und diese beidseitig zur Plattenober- und-unterseite überragt.

In Abhängigkeit der Anzahl der innerhalb des Kopfteils vorhandenen elektrisch leitenden Kontaktelemente ist eine entsprechende Anzahl, die Fixierplatte durchragende elektrische Leiterstrukturen vorzusehen. Hierzu werden die, die Fixierplatte orthogonal zur Plattenober- und -unterseite durchragenden elektrischen Leiterstrukturen unter Maßgabe der räumlichen Anordnung der elektrisch leitenden Kontaktelemente innerhalb des Kopfteils in der Fixierplatte verteilt angeordnet. Alternativ zum Einbetten der elektrischen Leiterstrukturen im Rahmen des Gießprozesses, bietet es sich ebenso an, die Fixierplatte separat zu elektrischen Leiterstrukturen im Wege des Gießprozesses herzustellen. Die elektrischen Leiterstrukturen werden in diesem Fall nachfolgend in feine, die Fixierplatte durchragende Bohrungen eingesetzt und mit einem Adhäsivkleber, bspw. mit einem Tropfen Epoxid benetzt, welches beim Aushärten die Leiterstrukturen fixiert und aus dem gleichen Material wie das Kopfteil besteht.

Zur Herstellung des Versorgungsteils wird in an sich bekannter Weise eine Gießform verwendet, die die äußere Form und Gestalt des Versorgungsteils bestimmt. Zur Vorbereitung des Gießprozesses wird die wenigstens eine elektrische Komponente , vorzugsweise in Form eines Mikrocontrollers und/oder einer elektrischen Energiequelle im Inneren der Gießform platziert und mit der wenigstens einen elektrischen Leiterstruktur verbunden. Lösungsgemäß ist die Gießform derart ausgebildet, so dass die als Halbzeug vorbereitete Fixierplatte in die Gießform integriert wird und mit ihrer Plattenunterseite eine die Gießform begrenzende Teiloberfläche bildet. Nachfolgend werden die wenigstens eine, die Plattenunterseite überragende elektrische Leiterstruktur sowie die wenigstens eine elektrische Komponente mit der aushärtbaren Vergussmasse vergossen.

Im Rahmen des Gießvorganges füllt die Vergussmasse die vorbereitete Gießform aus und umschließt die darin angeordneten Komponenten, wobei die fließfähige Vergussmasse die als Gießformteiloberfläche dienende Plattenunterseite der Fixierplatte unter Ausbildung einer monolithischen Stoffschlussverbindung benetzt.

Nach Aushärten der Vergussmasse wird das Versorgungsteil gemeinsam mit der fest am Versorgungsteil anhaftenden bzw. mit dieser fest verbundenen Fixierplatte aus der Gießform entnommen.

Zur Herstellung des Kopfteils gilt es zunächst das wenigstens eine, elektrisch leitende Kontaktelement vorzusehen und mit der wenigstens einen über die Plattenoberseite der Fixierstruktur ragenden elektrischen Leiterstruktur zu verbinden.

Üblicherweise sieht das Kopfteil eine Vielzahl elektrisch leitender Kontaktelemente vor, die in Form von Kontaktringelementen ausgebildet sind und sich in koaxialer und in axial serieller Abfolge mit elektrisch isolierenden, elastisch verformbaren Dichtungsringen abwechseln und unter axialer Fügekraft gegeneinander kraftschlüssig gefügt werden. Eine bevorzugte Ausbildung einer derartig vorgespannten Stapelanordnung von Kontaktringelementes, die in einem Kopfteil integriert ist, wird unter Bezugnahme der nachfolgenden Figuren näher erläutert.

Die über die Plattenoberseite der Fixierplatte ragenden elektrischen Leiterstrukturen werden mit den jeweils innerhalb des Kopfteils vorgesehenen elektrischen Kontaktelementen vorzugsweise im Wege eines Löt-, Bond-, Spaltschweiß- oder Reibschweißverfahrens elektrisch kontaktiert. Optional können im Kopfteil weitere elektrische Strukturen eingebracht werden, bspw. eine Antenne, die mit entsprechenden elektrischen Komponenten innerhalb des Versorgungsteils verbunden sein können. Im Weiteren wird das wenigstens eine mit der elektrischen Leiterstruktur elektrisch verbundene Kontaktelement mit der aushärtbaren Vergussmasse derart vergossen, so dass sich eine Stoffschlussverbindung zur Plattenoberseite der Fixierplatte ausbildet. Der Gießvorgang zur Herstellung des Kopfteils kann vor, während oder nach der Herstellung des Versorgungsteils durchgeführt werden.

Gleichsam wie im Falle des Gießvorganges zur Herstellung des Versorgungsteils bildet sich auch zwischen der Plattenoberseite der Fixierplatte und der kopfteilseitigen, aushärtbaren Vergussmasse eine fluiddichte, monolithische Stoffschlussverbindung aus, so dass sämtliche elektrische Komponenten, die mit Hilfe der aushärtbaren Vergussmasse im Bereich des Kopfteils, der Fixierplatte sowie des Versorgungsteils umgossen werden vollumfänglich fluiddicht von der Vergussmasse ohne jegliche Grenzflächen umgeben sind.

Als Vergussmasse zur Herstellung des Kopfteils, der Fixierplatte sowie auch des Versorgungsteils eignen sich biokompatible Kunststoffe oder Epoxidharze.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Darstellung eines lösungsgemäß ausgebildeten medizinischen Implantats,
- Fig. 2: Darstellung einer Fixierplatte mit durchragenden elektrischen Leiterstrukturen,
- Fig. 3: Gießform zur Herstellung des Versorgungsteil,
- Fig. 4: elektrische Verbindung der elektrischen Leiterstrukturen mit einer Anordnung aus Kontaktringelementen sowie
- Fig. 5: Darstellung zur Herstellung der Vergussmasse des Kopfteils

### Wege zur Ausführung der Erfindung, gewerbliche Verwendbarkeit

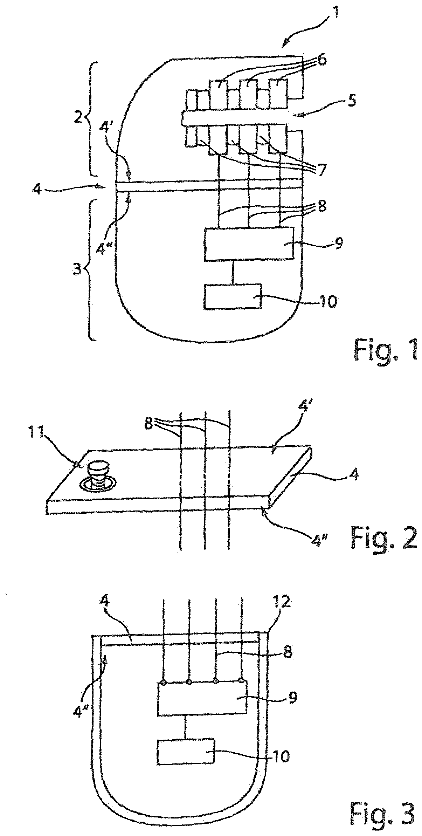

Fig. 1 zeigt ein lösungsgemäß hergestelltes medizinisches Implantat 1 umfassend ein Kopfteil 2, ein Versorgungsteil 3 sowie eine Fixierplatte 4, deren Plattenoberseite 4' mit dem Kopfteil 2 und deren Plattenunterseite 4" mit der Versorgungseinheit 3 stoffschlüssig, d.h. monolithisch verbunden sind.

Das Kopfteil 2 weist eine sacklochartige Ausnehmung 5 in Art einer elektrischen Steckkontaktbuchse auf, längs der in serieller Abfolge elektrische Kontaktelemente 6 in Form von Kontaktringelementen, jeweils axial beabstandet durch zwischenliegende elektrisch isolierende Dichtungsringe 7. Die serielle Abfolge aus Kontaktringelementen 6 und Dichtungsringen 7 unterliegt einer axialen Fügekraft F, durch die die Kontaktringelemente 6 und die Dichtungsringe 7 fluiddicht miteinander abschließen. Zur elektrischen Signal- und Energieversorgung sind die elektrischen Kontaktringelemente 6 über drahtartig ausgebildete elektrische Leiterstrukturen 8 mit einem innerhalb des Versorgungsteils 3 eingebrachten Mikrocontrollers 9 sowie einer mit dem Mikrocontroller 9 in elektrischer Verbindung stehenden elektrische Energiequelle 10 verbunden. Die elektrische Energiequelle 10 kann als Batterie, Akku, Biobrennstoffzelle oder in Form einer induktiven Koppelschleife zur kontaktlosen induktiven Energieübertragung ausgebildet sein. Selbstverständlich können alternative oder weitere elektrische Komponenten im Versorgungsteil 1 enthalten sein.

Sämtliche Komponenten des medizinischen Implantats 1 sind jeweils mit einer einheitlichen biokompatiblen Vergussmasse, vorzugsweise einer Kunststoff- oder Harzmasse, vorzugsweise Epoxidharzmasse, vergossen.

Zur Herstellung des in Fig. 1 illustrierten medizinischen Implantats 1 wird gemäß Fig. 2 die Fixierplatte 4 im Rahmen eines Gießverfahrens aus einer biokompatiblen Vergussmasse hergestellt, wobei die drahtförmigen elektrischen Leiterstrukturen 8 die Fixierplatte 4 orthogonal zu deren Plattenober- 4' sowie Plattenunterseite 4" durchragen. Optional ist innerhalb der Fixierplatte 4 ein mechanisches Verbindungsmittel 11 eingebracht, vorzugsweise in Form einer Gewinde-Mutter mit einer darin einbringbaren Schraube, zur ortsfesten Fixierung der Kopfteil-seitigen seriellen Abfolge aus Dichtungsringen 7 sowie Kontaktringelementen 6, siehe hierzu auch die Kontaktring-/Dichtungsringelement-Anordnung 14 in Figur 14, die mit einer entsprechenden mechanischen, nicht dargestellten Halterung für eine zusätzliche Fixierung an der Fixierplatte 4 vermittels einer Schraubverbindung 11 ausgestattet sein kann.

Die als separates Halbzeug ausgebildete Fixierplatte 4 mit den darin befestigten, drahtförmigen elektrischen Leiterstrukturen 8 dient im Weiteren als Teiloberfläche einer Gießform 12 zur Herstellung und Ausbildung des Versorgungsteils 3. Zunächst gilt es die über die Plattenunterseite 4" ragenden elektrischen Leiterstrukturen 8 mit dem dargestellten Mikrocontroller 9 sowie der elektrischen Energiequelle 10 elektrisch zu verbinden. Die elektrische Verbindung kann vor oder nach dem Einlegen der Fixierplatte 4 in die Gießform zu deren Begrenzung erfolgen.

Fig. 3 zeigt die in die Gießform 12 eingesetzte Fixierplatte 4, deren Plattenunterseite 4" das Innere der Gießform 12 fluiddicht zu begrenzen vermag. Im Nachfolgenden wird die Gießform 12 mit einer biokompatiblen aushärtbaren Vergussmasse vollständig verfüllt. Die beim Verfüllen der Gießform 12 verwendete biokompatible aushärtbare Vergussmasse ist identisch mit der, aus der die Fixierplatte 4 besteht, so dass sich eine stoffschlüssige, monolithische Verbindung zwischen der Plattenunterseite 4" der Fixierplatte 4 und der Vergussmasse ausbildet.

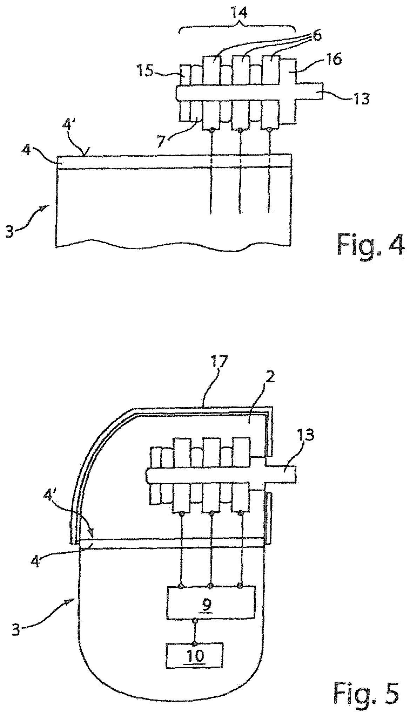

Im Anschluss an den Gießvorgang erfolgt gemäß Fig. 4 die elektrische Kontaktierung der über die Plattenoberseite 4' der Fixierplatte 4 ragenden elektrischen Leiterstrukturen 8 mit den Kontaktringelementen 6.

Die in Fig. 4 illustrierte axiale Stapelanordnung 14 bestehend aus elektrischen Kontaktringelementen 6 sowie dazwischen befindlichen Dichtungsringen 7 stellt gleichsam ein Halbzeug dar, mit einem stabförmigen ausgebildeten Montagewerkzeug 13, längs dem in axial abwechselnder Abfolge die elektrisch leitenden Kontaktringelemente 6 sowie jeweils dazwischen befindliche elektrisch isolierende, aus elastomeren Material gefertigte Dichtungsringe 7 angeordnet sind. Beidseitig zu der aus der abwechselnden Abfolge aus Kontaktringelementen 6 und Dichtungsringen 7 zusammengesetzten, axialen Stapelanordnung 14 sind jeweils längs des stabförmigen Montagewerkzeuges 13 Befestigungsmittel 15 und 16 angebracht. Im Falle des in Fig. 4 dargestellten Befestigungsmittels 16 handelt es sich um einen mit dem ansonsten stabförmig ausgebildeten Montagewerkzeug 13 einstückig verbundenen und teller- bzw. scheibenartig ausgebildeten mechanischen Anschlag, an den die axiale Stapelanordnung 14 einseitig unmittelbar angrenzt. Das zu diesem längs der axialen Stapelanordnung 14 gegenüberliegend angeordnete Befestigungsmittel 15 ist axial beweglich längs des stabförmigen Montagewerkzeuges 13 ausgebildet und verfügt überdies über einen Arretierungsmechanismus, der das Befestigungsmittel 15 axial fest relativ zum stabförmigen Montagewerkzeug zu fixieren vermag. Vorzugsweise stellt das Befestigungsmittel 15 eine Mutter oder eine Platte mit Innengewinde dar, das im Eingriff mit einem endseitig längs des stabförmigen Montagewerkzeuges 13 angebrachten Außengewindes, nicht dargestellt, steht.

Zum Aufbringen der axial zum stabförmigen Montagewerkzeug 13 orientieren Fügekraft, die die abwechselnde Abfolge von Kontaktringelementen 6 und Dichtungsringen 7 kraftschlüssig mit- bzw. gegeneinander fügt, gilt es das Montagewerkzeug 13 relativ zu dem Befestigungsmittel 15 zu drehen, beispielsweise durch vollständiges Eindrehen des montagewerkzeugseitigen Außengewindes in das Innengewinde des Befestigungsmittels 15, wodurch sich eine längs der auf dem Montagewerkzeug 13 aufsitzenden Kontaktringelementen 6 und Dichtungsringen 7 wirkende, definiert vorgebbare Fügekraft einstellt.

Die in Fig. 4 dargestellte axiale Stapelanordnung 14 stellt ein separat handzuhabendes Halbzeug dar, das nach entsprechender elektrischer Kontaktierung der elektrischen Leiterstrukturen 8 mit den elektrischen Kontaktringelementen 6 in eine Gießform 17 gemäß Fig. 5 eingelegt wird. Die Gießform 17 wird, gleichsam wie im Falle des Gießvorganges gemäß Fig. 3, von der einstückig mit der Versorgungsteil-seitigen Vergussmasse verbundenen Fixierplatte 4 teilweise begrenzt, d.h. die Plattenoberseite 4' der Fixierplatte 4 schließt fluiddicht mit der übrigen Gießform 17 ab. Der Gießprozess erfolgt in gleicher Weise unter Verwendung der erstarrungsfähigen Vergussmasse, aus der bereits die Fixierplatte 4 sowie der Gießkörper des Versorgungsteils 3 bestehen.

Nach Erstarren der Kopfteil-seitigen Vergussmasse kann das medizinische Implantat 1 aus der Gießform 17 entnommen werden und das Montagewerkzeug 13 durch Drehen von dem Befestigungsmittel 15 separiert werden. Die axial zwischen den Kontaktringelementen 6 und den Dichtungsringen 7 herrschende Fügekraft wird von der Kopfteil-seitig erstarrten Vergussemasse abgestützt und konserviert.

Alternativ zu der vorstehend erläuterten Verfahrensweise ist es gleichsam möglich, das Gießverfahren gemäß Fig. 5 zeitlich vor dem Gießverfahren zur Herstellung des Versorgungsteils 3 gemäß Fig. 3 oder zeitgleich mit diesem durchzuführen.

### Bezugszeichenliste

- 1: Medizinisches Implantat
- 2: Kopfteil
- 3: Versorgungsteil
- 4: Fixierplatte
- 4': Plattenoberseite
- 4": Plattenunterseite
- 5: sacklochartige Ausnehmung
- 6: elektrisch leitendes Kontaktelement, Kontaktringelement
- 7: Dichtungsring
- 8: elektrische Leiterstruktur
- 9: Mikrocontroller
- 10: elektrische Energiequelle, Batterie
- 11: mechanisches Verbindungsmittel
- 12: Gießform
- 13: Montagewerkzeug
- 14: Stapelanordnung
- 15, 16: Befestigungsmittel
- 17: Gießform

## Patentansprüche

1. Verfahren zum Herstellen eines medizinischen Implantats (1), umfassend ein Kopfteil (2), das wenigstens eine sacklochartige Ausnehmung (5) in Art einer elektrischen Steckkontaktbuchse besitzt, längs der wenigstens ein elektrisch leitendes Kontaktelement (6) angeordnet ist, sowie ein mit dem Kopfteil (2) fest verbundenes Versorgungsteil (3), das wenigstens eine elektrische Komponente umfasst, die mit dem wenigstens einen elektrisch leitenden Kontaktelement (6) über wenigstens eine elektrische Leiterstruktur (8) elektrisch verbunden sind,
**gekennzeichnet durch** folgende Verfahrensschritte:
- Herstellen einer aus einer aushärtbaren Vergußmasse bestehenden, eine Plattenober- und -unterseite (4`,4") aufweisenden Fixierplatte (4), in der die wenigstens eine elektrische Leiterstruktur (8) fixiert wird und dabei die Plattenober- und -unterseite (4', 4") durchragt,
- Anordnen der Fixierplatte (4) in einer Gießform (12) derart, dass die Plattenunterseite (4") eine Teiloberfläche der Gießform (12) bildet,
- Elektrisches Kontaktieren der wenigstens einen elektrischen Leiterstruktur (8) mit der wenigstens einen elektrischen Komponente, vor oder nach dem Anordnen der Fixierplatte (4) als die Gießform (12) teilbegrenzende Teiloberfläche, und
- Herstellen des Versorgungsteils (3) durch Umgießen des wenigstens einen elektrischen Komponente sowie der wenigstens einen, die Plattenunterseite (4") überragenden elektrischen Leiterstruktur (8) mittels der aushärtbaren Vergußmasse unter Ausbildung einer Stoffschlussverbindung mit der Plattenunterseite (4") der Fixierplatte (4).

2. Verfahren nach Anspruch 1,
**dadurch gekennzeichnet, dass** das Herstellen der Fixierplatte (4) im Wege eines Gießverfahrens erfolgt, bei dem die aushärtbare Vergußmasse in eine Plattengießform eingebracht wird.

3. Verfahren nach Anspruch 2,
**dadurch gekennzeichnet, dass** die Gießform (12) von der wenigstens einen elektrischen Leiterstruktur (8) durchragt wird, während der Gießvorgang durchgeführt wird.

4. Verfahren nach Anspruch 3,
**dadurch gekennzeichnet, dass** in die ausgehärtete Fixierplatte (4) wenigstens ein Loch eingebracht wird, durch das die elektrische Leiterstruktur (8) geführt wird und mittels eines Adhäsivklebers in dem Loch fixiert wird.

5. Verfahren nach einem der Ansprüche 1 bis 4,
**dadurch gekennzeichnet, dass** die wenigstens eine über die Plattenoberseite (4') ragende elektrische Leiterstruktur (8) mit dem wenigstens einen elektrisch leitenden Kontaktelement (6) elektrisch verbunden wird, und
dass das wenigstens eine elektrisch leitende Kontaktelement (6) mittel- oder unmittelbar mit der Fixierplatte (4) fest verbunden wird.

6. Verfahren nach einem der Ansprüche 1 bis 5,
**dadurch gekennzeichnet, dass** eine Vielzahl der elektrisch leitenden Kontaktelemente (6) vorgesehen wird, die in Form von Kontaktringelementen (6) ausgebildet sind, sich in koaxialer und in axial serieller Abfolge mit elektrisch isolierenden, elastisch verformbaren Dichtungsringen (7) abwechseln und unter axialer Fügekraft gegeneinander kraftschlüssig gefügt werden, und
dass die Vielzahl der in koaxialer und in axial serieller Abfolge angeordneten Kontaktringelemente (6) und Dichtungsringe (7), nach elektrischer Kontaktierung der Kontaktringelemente (6) jeweils mit wenigstens einer über die Plattenoberseite (4') ragenden elektrischen Leiterstruktur (8), fest mit der Plattenoberseite (4') der Fixierplatte (8) verbunden wird.

7. Verfahren nach Anspruch 5 oder 6,
**dadurch gekennzeichnet, dass** vor, während oder nach der Herstellung des Versorgungsteils (3) das wenigstens eine mit der elektrischen Leiterstruktur (8) elektrisch verbundene, elektrisch leitende Kontaktelement (6) mit der aushärtbaren Vergußmasse unter Ausbildung des Kopfteils (2) derart vergossen wird, dass sich eine Stoffschlussverbindung zur Plattenoberseite (4') der Fixierplatte (4) ausbildet.

8. Verfahren nach einem der Ansprüche 1 bis 7,
**dadurch gekennzeichnet, dass** als Vergußmasse biokompatibles Polymer, insbesondere ein biokompatibles Epoxid-Harzmasse verwendet wird.

9. Verfahren nach einem der Ansprüche 1 bis 8,
**dadurch gekennzeichnet, dass** das Versorgungsteil (3) als als Pulsgenerator dienende implantierbare medizinische Vorrichtung ausgebildet wird.

## Claims

1. Method for the production of a medical implant (1), comprising a head section (2), which has at least one blind hole-type recess (5) in the manner of an electrical plug-in contact socket, along which is arranged at least one electrically conductive contact element (6), and a supply section (3), which is fixedly connected to the head section (2) and comprises at least one electrical component, which is electrically connected to the at least one electrically conductive contact element (6) by way of at least one electrical conductor structure (8),
**characterised by** the following steps of the method:
- Production of a fixing plate (4), consisting of a curable casting compound, and having a plate upper face and a plate lower face (4', 4"), in which the at least one electrical conductor structure (8) is fixed, and at the same time projects through the plate upper and lower faces (4', 4"),
- Arrangement of the fixing plate (4) in a casting mould (12), that the plate lower face (4") forms part of the surface of the casting mould (12),
- Establishment of electrical contact between the at least one electrical conductor structure (8) and the at least one electrical component, before or after the arrangement of the fixing plate (4) as part of the surface partially bounding the casting mould (12), and
- Production of the supply section (3) by the encapsulation of the at least one electrical component, and the at least one electrical conductor structure (8) projecting beyond the lower face (4") of the plate, in the curable casting compound, with the formation of a material bond with the lower face (4") of the fixing plate (4).

2. Method according to Claim 1,
**characterised in that**, the fixing plate (4) is produced by way of a casting method, in which the curable casting compound is introduced into a plate casting mould.

3. Method according to Claim 2,
**characterised in that**, the casting mould (12) is penetrated by the at least one electrical conductor structure (8), while the casting process is being carried out.

4. Method according to Claim 3,
**characterised in that**, at least one hole is introduced into the cured fixing plate (4), through which the electrical conductor structure (8) is passed, and is fixed in the hole by means of an adhesive.

5. Method according to one of the Claims 1 to 4,
**characterised in that**, the at least one electrically conductive structure (8), projecting beyond the plate upper face (4'), is electrically connected to the at least one electrically conductive contact element (6),
and **in that**, the at least one electrically conductive contact element (6) is fixedly connected, indirectly or directly, to the fixing plate (4).

6. Method according to one of the Claims 1 to 5,
**characterised in that**, a multiplicity of electrically conductive contact elements (6) is provided, which are designed in the form of contact ring elements (6), alternate in a coaxial, and in an axial serial, sequence with electrically insulating, elastically deformable, sealing rings (7), and are joined in a force fit with one another under an axial clamping force,
and **in that**,
the multiplicity of contact ring elements (6) and sealing rings (7) arranged in a coaxial, and in an axially serial, sequence, after electrical contact has been established between the contact ring elements (6) in each case with at least one electrical conductor structure (8) projecting beyond the plate upper face (4'), are fixedly connected to the upper face (4') of the fixing plate (8).

7. Method according to Claim 5 or 6,
**characterised in that**, before, during, or after, the production of the supply section (3), the at least one electrically conductive contact element (6), which is electrically connected to the electrical conductor structure (8), is encapsulated in the curable casting compound, forming the head section (2), such that a material bond is formed with the upper face (4') of the fixing plate (4).

8. Method according to one of the Claims 1 to 7,
**characterised in that**, a biocompatible polymer, in particular a biocompatible epoxy resin, is used as the casting material.

9. Method according to one of the Claims 1 to 8,
**characterised in that**, the supply section (3) is designed as an implantable medical device serving as a pulse generator.

## Revendications

1. Procédé de fabrication d'un implant médical (1) comprenant une pièce de tête (2), qui possède au moins un évidement (5) en forme de trou borgne à la manière d'une douille de connexion enfichable électrique, le long de laquelle est disposé au moins un élément de contact électroconducteur (6), ainsi qu'une partie d'alimentation (3) fermement reliée à la pièce de tête (2), qui comprend au moins un composant électrique qui sont reliés électriquement à au moins un élément de contact électroconducteur (6) par au moins une structure conductrice électrique (8),
**caractérisé par** les étapes de procédé suivantes :
- fabrication d'une plaque de fixation (4) composée d'une masse de remplissage durcissable, comportant une face supérieure et une face inférieure de plaque (4', 4"), dans laquelle est fixée au moins une structure conductrice électrique (8) et traverse à cet effet la face supérieure et la face inférieure de plaque (4', 4"),
- mise en place de la plaque de fixation (4) dans un moule (12) de telle manière que la face inférieure de plaque (4'') forme une partie de surface du moule (12),
- mise en contact électrique d'au moins une structure conductrice électrique (8) avec au moins un composant électrique avant ou après la mise en place de la plaque de fixation (4) en tant que partie de surface limitant partiellement le moule (12) et
- fabrication de la partie d'alimentation (3) par surmoulage d'au moins un composant électrique ainsi que d'au moins une structure conductrice électrique (8) dépassant la face inférieure de plaque (4") au moyen de la masse de remplissage durcissable en formant une liaison par conformité de matière avec la face inférieure de plaque (4") de la plaque de fixation (4).

2. Procédé selon la revendication 1,
**caractérisé en ce que** la fabrication de la plaque de fixation (4') a lieu au cours d'un procédé de coulée lors duquel la masse de remplissage durcissable est introduite dans un moule de plaque.

3. Procédé selon la revendication 2,
**caractérisé en ce que** le moule (12) est traversé par au moins une structure conductrice électrique (8), pendant que l'opération de coulée est exécutée.

4. Procédé selon la revendication 3,
**caractérisé en ce qu'au** moins un trou est aménagé dans la plaque de fixation (4) durcie à travers lequel la structure conductrice électrique (8) est passée et est fixée dans le trou au moyen d'un agent adhésif.

5. Procédé selon l'une quelconque des revendications 1 à 4,
**caractérisé en ce qu'au** moins une structure conductrice électrique (8) dépassant sur la face supérieure de plaque (4') est électriquement reliée au moins à un élément de contact électroconducteur (6) et
**en ce qu'au** moins un élément de contact électroconducteur (6) est fermement relié indirectement ou directement à la plaque de fixation (4).

6. Procédé selon l'une quelconque des revendications 1 à 5,
**caractérisé en ce qu'une** pluralité d'éléments de contact électroconducteurs (6) est prévue, lesquels sont constitués sous la forme d'éléments annulaires de contact (6), alternant en succession coaxiale et axiale avec des bagues d'étanchéité (7) électro-isolantes, élastiquement déformables et sont assemblés par conformité de force l'un contre l'autre par force d'assemblage axiale et
**en ce que** les éléments annulaires de contact (6) et bagues d'étanchéité (7) en pluralité disposés en succession coaxiale et axialement en série sont reliés fermement à la face supérieure de plaque (4') de la plaque de fixation (8) après mise en contact électrique des éléments annulaires de contact (6) respectivement avec au moins une structure conductrice électrique (8) dépassant sur la face supérieure de plaque (4').

7. Procédé selon la revendication 5 ou 6, **caractérisé en ce qu'avant,** pendant ou après la fabrication de la partie d'alimentation (3), au moins un élément de contact électroconducteur (6) relié électriquement à la structure conductrice électrique (8) est coulé avec la masse de remplissage durcissable en formant la partie de tête (2) de telle manière qu'une liaison par conformité de matière est constituée vers le côté supérieur de plaque (4') de la plaque de fixation (4).

8. Procédé selon l'une quelconque des revendications 1 à 7, **caractérisé en ce que** du polymère biocompatible, en particulier une masse de résine époxy biocompatible, est utilisé comme masse de remplissage.

9. Procédé selon l'une quelconque des revendications 1 à 8, **caractérisé en ce que** la partie d'alimentation (3) est constituée comme dispositif médical implantable servant de générateur d'impulsions.
